Europäisches Patentamt

European Patent Office

Office européen des brevets

(19)

(11) Numéro de publication: **0 212 998**
**A2**

(12) # DEMANDE DE BREVET EUROPEEN

(21) Numéro de dépôt: **86401389.1**

(22) Date de dépôt: **24.06.86**

(51) Int. Cl.⁴: **A 61 K 31/135**

---

(30) Priorité: **24.06.85 FR 8509572**

(43) Date de publication de la demande: **04.03.87**
**Bulletin 87/10**

(84) Etats contractants désignés: **AT BE CH DE FR GB IT LI LU NL SE**

(71) Demandeur: **Albert ROLLAND S.A. Société dite, 49, Rue St-André-des-Arts, F-75006 Paris (FR)**

(72) Inventeur: **Rolland, Anne, 49, rue Lamarck, F-75018 Paris (FR)**

(74) Mandataire: **Burtin, Jean-François, 5 Bis, rue Parmentier, F-92200 Neuilly S/Seine (FR)**

---

(54) **Utilisation de la médifoxamine pour l'amélioration des performances psychomotrices.**

(57) L'invention se rapporte au domaine de la chimie pharmaceutique et plus particulièrement au domaine de la thérapeutique.

Elle concerne des compositions pharmaceutiques améliorant les performances psychomotrices qui renferment, à titre de principe actif, une quantité neurologiquement active de 2,2-bis phénoxy NN-dimethyléthylamine en association ou en mélange avec un excipient ou un véhicule pharmaceutique.

Les compositions de l'invention trouvent un emploi thérapeutique dans le traitement de la fatigue intellectuelle.

EP 0 212 998 A2

## NOUVELLES COMPOSITIONS PHARMACEUTIQUES AMELIORANT LES PERFORMANCES
## PSYCHOMOTRICES ET LEUR PROCEDE D'OBTENTION

L'invention concerne de nouvelles compositions pharmaceutiques destinées à corriger ou à améliorer les performances psychomotrices.

Elle a plus particulièrement pour objet de nouvelles compositions pharmaceutiques destinées à améliorer la vigilance ou à combattre les effets liés à la fatigue intellectuelle.

Spécifiquement, l'invention se rapporte à des compositions pharmaceutiques renfermant à titre de principe actif une quantité neurologiquement activé de 2,2-bis-phénoxy-N,N-diméthyléthylamine ou Medifoxamine, ou un de ses sels d'addition avec un acide minéral ou organique en association ou en mélange avec un excipient ou un véhicule inerte non toxique pharmaceutiquement acceptable.

On a constaté, en effet, d'une manière surprenante que, contrairement aux effets habituels des anti-dépresseurs ou des anxiolytiques, la Medifoxamine, non seulement ne provoque pas de baisse de l'acuité intellectuelle mais encore est susceptible de corriger ou d'améliorer les performances psychomotrices du sujet sain.

On sait que parmi les effets des substances neurotropes comme les agents tranquillisants ou psychotropes comme les agents anti-dépresseurs, un des effets secondaires les plus gênants est la perte d'attention ou la baisse de l'acuité intellectuelle. C'est ainsi que les dictionnaires médicaux citent à titre de précaution d'emploi le risque de somnolence pour les conducteurs de machine ou les conducteurs de véhicule, ainsi que la diminution de la capacité de concentration.

Le Dictionnaire Vidal en particulier mentionne pour la plupart des médicaments anti-dépresseurs ou anxiolytiques, la précaution d'emploi suivante: ce médicament peut affaiblir les facultés mentales et physiques nécessaires à l'exécution de certaines tâches dangereuses telles que la manipulation d'appareils ou la conduite d'un véhicule à moteur, risque de somnolence.

Il est donc important dans le traitement de l'anxiété ou des psychoses dépressionnelles ou maniaco-involutives, qui nécessite une administration prolongée, de disposer d'un médicament qui pallie ces effets gênants et permet ainsi de maintenir une activité professionnelle normale.

Il est également important de pouvoir administrer à des sujets normaux présentant une cause de fatigabilité intellectuelle importante, un médicament permettant de maintenir les performances psychomotrices à un niveau normal. C'est le cas par exemple des employés travaillant la nuit, des services de garde, des ouvrières effectuant des travaux nocturnes.

Les compositions selon l'invention sont administrées par voie parentérale sous forme d'ampoules, de flacons multidoses, de seringues auto-injectables; par voie buccale sous forme de comprimés, de comprimés enrobés, de dragées, de microgranules, de gélules, de capsules, de pilules, de gouttes, de solutions ou de suspensions buvables ou encore par voie rectale sous forme de capsules rectales ou de suppositoires.

Les excipients ou véhicules qui conviennent le mieux pour de telles formes d'administration sont l'eau ou les liquides aqueux salins, les amidons, la cellulose microcristalline, le phosphate tricalcique, le phosphate de magnésium, le stéarate de magnésium, le talc, les carboxy-méthylcellulose ou les polyvinylpyrrolidones; le beurre de cacao ou les stéarates de polyéthylèneglycols.

La posologie usuelle s'échelonne de 40 à 200 mg de Medifoxamine par prise unitaire et de préférence de 40 à 60 mg de Medifoxamine. La posologie journalière s'échelonne de 200 à 400 mg par jour répartie en une à quatre prises.

La posologie peut varier dans des limites sensibles en fonction du poids du sujet, de l'âge du sujet, de l'indication thérapeutique et de la voie d'administration.

L'invention comprend également un procédé d'obtention des compositions pharmaceutiques améliorant les performances psychomotrices qui consiste en ce qu'on incorpore ou on ajoute à une quantité active de Medifoxamine ou à un de ses sels d'addition avec un acide minéral ou organique, un ou plusieurs excipients ou véhicules inertes, non toxiques, pharmaceutiquement acceptables.

Les exemples suivants illustrent l'invention sans toutefois la limiter.

## EXEMPLE I

Comprimés renfermant 50 mg de Medifoxamine sous forme de fumarate:

| | |
|---|---|
| . fumarate de Medifoxamine ............ | 70,4 g |
| . amidon de blé ...................... | 45 g |
| . amidon de maïs ..................... | 60 g |
| . cellulose microcristalline .......... | 5 g |
| . caséine formolée ................... | 4,6 g |
| . stéarate de magnésium .............. | 12 g |
| . talc ............................... | 3 g |

pour mille comprimés terminés à 0,200 g.

## EXEMPLE II

## ETUDE DES EFFETS DE LA MEDIFOXAMINE A 50 mg SUR LA PERFORMANCE PSYCHO-MOTRICE

Une expérimentation a été conduite dans le but d'évaluer les effets d'une administration de MEDIFOXAMINE à 50 mg à dose répétées pendant quatre jours, sur des tâches d'attention et de vigilance et sur des tests de mémoire immédiate, permettant d'évaluer la performance psychomotrice d'un opérateur humain.

## Méthodologie

## Evaluation de la performance psychomotrice

La performance humaine a été mesurée, d'une part, à l'aide d'une tâche psychomotrice complexe associant une tâche principale de poursuite visuelle compensée sur deux axes, appelée tracking et une tâche secondaire de mesure des temps de réaction à un stimulus visuel et d'autre part, grâce à un test mettant en jeu la mémoire immédiate, décrit par De FAYOLLE et appelé VIGIL 97.

### Tracking:

Les informations visuelles à l'origine de la tâche principale de poursuite sont présentées sur un cadran dérivé d'un système aéronautique d'aide à l'atterrissage par mauvaise visibilité (ILS: Instrument Landing System). Ce cadran à deux aiguilles croisées est placé sur un bâti métallique, face à l'opérateur et à environ 0,7 m de ses yeux.

Les aiguilles qui correspondent chacune à un axe horizontal et vertical se déplacent selon un programme préenregistré sur bande magnétique.

Il s'agit de deux sinusoïdes de tension crête constante mais de fréquence variable, comprise entre 0,1 et 0,05 Hz et déphasées entre elles.

Au cours du test, dont la durée est fixée à 10 mn, le sujet doit maintenir les deux aiguilles orthogonales au centre de l'instrument par l'intermédiaire d'un manche de commande miniature placé devant lui sur une table.

Les écarts de position des deux aiguilles par rapport à l'axe vertical et à l'axe horizontal constituent les erreurs commises par le sujet.

Ces données sont traitées dans un calculateur analogique qui fournit par sommation des écarts, un score d'erreurs en unités machine pour la période de 10 mn après comparaison de la réponse du sujet et de la réponse idéale mise en mémoire.

Ce calcul d'erreur est effectué séparément pour le site (axe vertical ou localizer) et pour le gisement (axe horizontal ou glide).

Temps de réaction global :

Au cours du test de tracking, l'opérateur doit effectuer une tâche secondaire de détection et d'extinction de signaux lumineux. Il s'agit de deux lampes situées sur un axe horizontal au niveau des yeux du sujet et sur le même plan que le cadran ILS à 0,45 m de part et d'autre de celui-ci. La lampe gauche est rouge, la lampe droite est verte, elles s'allument indépendamment l'une de l'autre et de façon aléatoire.

Dès qu'un signal est perçu, le sujet doit appuyer sur un bouton de commande d'extinction placé sur une poignée gardée dans la main non occupée par le tracking.

Au cours du test de 10 mn qui est contemporain de la tâche de poursuite, 16 signaux sont présentés. L'intervalle de temps entre deux allumages successifs varie de façon aléatoire entre 25 et 60 secondes. Le temps séparant l'allumage d'une lampe de son extinction est mesuré avec une précision de l'ordre de ± 5 mS.

Ce temps est visualisé sur un voltmètre digital et imprimé automatiquement sur papier sous forme numérique. Pour chaque test est calculé un temps moyen d'extinction des feux colorés.

Test de mémorisation :

Le test Vigil 97 est un test de mémorisation permettant d'apprécier l'empan moyen de la mémoire immédiate. Il est programmé et présenté sur un micro-calculateur de bureau HEWLETT PACKARD HP 97. Un nombre quelconque est présenté deux fois de suite au sujet pendant une seconde. Les deux affichages se suivent de façon quasi instantanée.

Dès l'instruction du deuxième affichage le sujet doit, à l'aide du clavier du calculateur, reproduire le nombre qui avait été affiché.

Si la réponse est correcte, un nouveau nombre quelconque incrémenté d'un chiffre sera alors présenté au sujet dans les mêmes conditions et ainsi de suite.

Au contraire, si la réponse est erronée, un nombre quelconque décrémenté d'un chiffre sera présenté.

Une séquence comporte la présentation de dix nombres différents. Il est demandé au sujet testé, d'exécuter deux séquences consécutives.

Pour chaque séquence le programme permet le calcul de l'empan moyen de la mémoire, exprimé en nombre de chiffres mémorisés et sa variabilité.

Protocole
---------

L'étude a porté sur douze sujets adultes de sexe mâle, âgés de 22 à 49 ans et dont le poids était compris entre 65 et 78 kg.

Les sujets volontaires et en bonne santé mènent habituellement une existence active, sont entraînés et familiarisés depuis longtemps aux tâches psychomotrices décrites ci-dessus.

Au cours de l'expérimentation conduite en monothérapie stricte, les sujets s'abstenaient de tout psycho-stimulant ou excitant le jour des tests, l'absorption d'alcool interdite pendant les séquences de traitement.

L'étude a été réalisée en double insu. Chaque sujet participant à l'étude a été son propre témoin et a reçu un numéro d'ordre correspondant à la randomisation des deux séquences de traitement établies, l'une correspondant à la MEDIFOXAMINE à 50 mg, l'autre à un placébo présenté sous le même conditionnement.

Chaque sujet a reçu, pendant trois jours:
- 2 comprimés de MEDIFOXAMINE à 50 mg ou de placébo avant le repas du matin;
- 2 comprimés de MEDIFOXAMINE à 50 mg ou de placébo avant le repas du midi;
- 2 comprimés de MEDIFOXAMINE à 50 mg ou de placébo avant le repas du soir;

puis le quatrième jour:

- 2 comprimés de MEDIFOXAMINE à 50 mg ou de placébo avant le repas du matin.

Les tests psychomoteurs étaient subis entre 1 h et 3 heures après la prise médicamenteuse unique du 4ème jour de traitement.

Chaque sujet a alternativement effectué deux séries de chaque test: avant l'administration de toute molécule, puis entre 1 h et 3 heures après la dernière administration de MEDIFOXAMINE à 50 mg ou de placébo.

Les tests passés par les sujets aux jours J1 et J15 avant la prise et aux jours 4 et 18 après la prise étaient identiques et consistaient successivement en deux tâches de tracking séparées et suivies d'un test de De FAYOLLE.

Méthodologie statistique

Le traitement statistique des résultats a fait appel à la théorie d'analyse de variance.

Sous la double hypothèse de la normalité des distributions et de l'indépendance entre moyennes et variances, les calculs de l'analyse de variance ont été effectués selon un plan en facteurs croisés.

Trois facteurs qualitatifs ont été utilisés. L'un d'eux correspond à l'organisation du protocole expérimental en blocs complets, un bloc étant identifié à un sujet. Il s'agit donc de l'effet "sujet".

Le second facteur décrit l'influence du médicament administré dans quatre situations différentes:

- 1er essai de référence;
- 2ème essai de référence;
- Placébo;
- MEDIFOXAMINE à 50 mg appelé "produit C".

Le dernier facteur enfin rend compte de l'évolution dans le temps de la performance psychomotrice au cours du test puisque deux batteries de test étaient proposées successivement au cours de chaque test. En d'autres termes, ce facteur décrit l'effet fatigue.

L'analyse permet, outre l'estimation de la signification des trois facteurs décrits, l'estimation de la signification du terme d'interaction entre les deux facteurs expérimentaux "médicament" et "fatigue". Ce test permet d'évaluer l'influence réciproque de ces deux facteurs l'un sur l'autre. Autrement dit, il permet de répondre à la question de savoir si la fatigue apparue au cours de l'essai est constante en fonction du "médicament" reçu.

Les autres tests statistiques restent inclus dans l'erreur aléatoire. Les résultats de ces différentes estimations sont donnés par le test F de Fischer-Snedecor.

Après l'estimation de signification de chacun des facteurs expérimentaux retenus, une analyse plus fine des résultats est effectuée sous la forme d'une comparaison des différentes moyennes deux à deux.

Le test choisi est le test t de Student ou le test g dit du "range studentisé" selon les conditions d'applications respectives de ces deux tests.

Le dénominateur de chacun de ces deux tests utilise l'erreur aléatoire telle qu'elle est calculée par l'analyse de variance.

Résultats

Test de tracking

Les valeurs numériques du test de tracking sont la somme des scores réalisés sur chacun des deux axes horizontaux et verticaux.

Les calculs montrent:

- un effet sujet très fortement significatif $F_{(11,77)} = 322$
- l'effet médicament est significatif au risque de 1 p. cent $F_{(3,77)} = 5,07$.

Les moyennes respectives, assorties d'un écart type égal à 6,9 sont égales à:

.  référence test 1 .................... 309,5
. référence test 2 .................... 309,5
. placébo ........................... 308,6
. produit C soit MEDIFOXAMINE ........ 293,4

Il existe des différences significatives deux à deux entre:

- la référence test 1 et la MEDIFOXAMINE t = 3,25
- la référence test 2 et la MEDIFOXAMINE t = 3,23
- la placébo et la MEDIFOXAMINE t = 3,06.

Par contre, les deux valeurs de référence et la moyenne des scores sous placébo sont très proches l'une de l'autre.

L'effet "fatigue" n'est pas significatif $F_{(1,77)} = 0,36$ et le test d'interaction "Médicament-temps" n'est pas, lui non plus, significatif $F_{(3,77)} = 0,07$.

Test de De FAYOLLE

Les données numériques de ce test ont été traitées selon le même procédé que précédemment, à ceci près que le facteur "fatigue" comprend

0212998

quatre niveaux correspondant aux quatre tests réalisés au cours du même essai (deux séquences et deux tests).

- l'effet "sujet" est très significatif: $F(11,165) = 10,93$;
- l'effet médicament est significatif au risque de 2 p. cent: $F(3,165) = 3,48$.

Les valeurs moyennes, assorties d'un écart type égal à 0,1 sont respectivement de:

. référence test 1 ............ 6,9
. référence test 2 ............ 7,1
. placébo ................... 7,0
. MEDIFOXAMINE à 50 mg ....... 7,1

Il n'existe pas de différence significative entre le Placébo et la MEDIFOXAMINE à 50 mg.

- l'effet "fatigue" n'est pas significatif: $F(3,165) = 0,34$;
- le test d'interaction "médicament-fatigue" n'est pas significatif $F(9,165) = 0,54$.

Conclusion

L'étude menée chez douze sujets adultes de sexe mâle a permis de montrer que l'administration de MEDIFOXAMINE à 50 mg pendant 3 jours à la dose quotidienne de 3 fois 2 comprimés n'entraîne aucun effet secondaire défavorable sur le niveau de vigilance d'un opérateur humain.

Au contraire, le test de poursuite compensé, considéré dans ce protocole comme la tâche psychomotrice principale est significativement amélioré par la MEDIFOXAMINE à 50 mg.

REVENDICATIONS

L'invention a pour objet:

1.- De nouvelles compositions pharmaceutiques améliorant les performances psychomotrices caractérisées en ce qu'elles renferment à titre de principe actif une quantité neurologiquement active de 2,2-bis-phénoxy-N,N-diméthyléthylamine ou un de ses sels d'addition avec un acide minéral ou organique en association ou en mélange avec un excipient ou un véhicule inerte non-toxique pharmaceutiquement acceptable.

2.- Des compositions pharmaceutiques selon la revendication 1, dans lesquelles la quantité neurologiquement active de principe actif s'échelonne de 40 à 2 00 mg de 2,2-bis-phénoxy-N,N-diméthyléthylamine.

3.- Des compositions pharmaceutiques selon l'une des revendications 1 ou 2, dans lesquelles l'excipient ou le véhicule est un de ceux qui conviennent pour l'administration par voie parentérale, buccale ou rectale.

4.- Un procédé d'obtention des compositions pharmaceutiques améliorant les performances psychomotrices selon l'une des revendications 1 à 3, caractérisé en ce que l'on incorpore ou additionne une quantité neurologiquement active de principe actif à un ou plusieurs excipients ou véhicules inertes, non toxiques pharmaceutiquement acceptables.